# EUROPÄISCHE PATENTSCHRIFT

(11) **EP 2 164 461 B1**
(45) Veröffentlichungstag und Bekanntmachung des Hinweises auf die Patenterteilung: **23.01.2013**
(21) Anmeldenummer: 08760353.6
(22) Anmeldetag: 02.06.2008
(51) Int. Cl.: A61K 47/32, A61K 9/00, A61K 9/20, A61K 47/26, A61K 47/34, A61K 47/36, A61K 47/38

(54) **KAU- UND LUTSCH-TABLETTEN**
CHEWABLE TABLETS AND LOZENGES
COMPRIMES A MACHER ET A SUCER

(30) Priorität: 06.06.2007 EP 07109713
(43) Veröffentlichungstag der Anmeldung: 24.03.2010
(73) Patentinhaber: BASF SE, 67056 Ludwigshafen (DE)
(72) Erfinder: KOLTER, Karl, 67117 Limburgerhof (DE); SCHÖNHERR, Michael, 67227 Frankenthal (DE); GEBERT, Silke, 67269 Grünstadt (DE); MEYER-BÖHM, Kathrin, 90537 Feucht (DE); MASCHKE, Angelika, 68159 Mannheim (DE)
(86) Internationale Anmeldenummer: PCT/EP2008/056766
(87) Internationale Veröffentlichungsnummer: WO 2008/148734

(56) Entgegenhaltungen:
- WO-A-98/22094
- WO-A-03/041683
- WO-A-2007/071581
- US-A1- 2001 016 208
- US-A1- 2002 071 864
- US-A1- 2005 244 343
- US-A1- 2005 244 492

## Beschreibung

Die vorliegende Erfindung betrifft pharmazeutische Formulierungen für die Herstellung von Kau- und Lutsch-Tabletten, enthaltend Agglomerate auf Basis von Zucker oder Zuckeralkoholen sowie Sprengmittel und wasserunlösliche Polymeren in Kombination mit einem viskositätserhöhenden/ gelbildenden Polymeren sowie die entsprechenden Kau- und Lutsch-Tabletten.

Kau- und Lutsch-Tabletten gewinnen für die orale Applikation von Arzneistoffen immer größere Bedeutung. Solche Tabletten müssen gut kaubar sein, angenehm schmecken und dürfen kein sandiges Gefühl hinterlassen. Ferner sollen sie einfach herstellbar sein, wobei die Direkttablettierung erhebliche Vorteile gegenüber der Feuchtgranulation bietet, und eine hohe mechanische Festigkeit besitzen, damit sie Verpackungsprozeduren, Transporte und auch das Herausdrücken aus Verpackungen unbeschadet überstehen.

Die bisher beschriebenen Produkte und Verfahren erfüllen diese Anforderungen nicht oder nur sehr unzureichend.

In der WO 2003/051338 ist eine direkttablettierbare und gut verpressbare Hilfsstoffformulierung, welche Mannit und Sorbit enthält, beschrieben. Zunächst wird durch Lösen von Mannit und Sorbit in Wasser und anschließender Sprühtrocknung (gewöhnliche Sprühtrocknung und SBD-Verfahren) eine Hilfsstoffvormischung hergestellt. Dieser coprozessierten Mischung kann zusätzlich Mannit zugesetzt werden. Tabletten, welche zusätzlich Sprengmittel, Trennmittel, Pigment und einen Wirkstoff enthalten, sollen innerhalb von 60 Sekunden in der Mundhöhle zerfallen.

In der US 2002/0071864 A1 wird eine Tablette beschrieben, welche innerhalb von 60 Sekunden in der Mundhöhle zerfällt, und hauptsächlich aus einer physikalischen Mischung von sprühgetrocknetem Mannit und einem grobkörnigen quervernetztem Polyvinylpyrrolidon sowie einer begrenzten Auswahl an Wirkstoffen formuliert ist. Diese Tabletten besitzen eine Bruchfestigkeit von ca. 40N und erzeugen ein unangenehmes, sandiges Mundgefühl.

Gemäß der US 6,696,085 B2 soll ein Methacrylsäure-Copolymer Typ C als Zerfallsmittel eingesetzt werden. Das Methacrylsäure-Copolymer Typ C ist ein magensaftresistentes Polymer, welches im sauren pH-Bereich nicht löslich ist, im pH-Bereich von 7, wie er in der Mundhöhle vorliegt, aber wasserlöslich ist. Die Tabletten weisen neben einer niedrigen Bruchfestigkeit (<20N) eine hohe Friabilität (>7%) auf und beinhalten einen hohen Anteil im Bereich von 15Gew.-% eines grobkörnigen Sprengmittels. Sie besitzen folglich eine niedrige mechanische Festigkeit und erzeugen aufgrund des hohen Anteils an grobkörnigem Sprengmittel ein unangenehmes, sandiges Mundgefühl.

EP 0839526 A2 beschreibt eine pharmazeutische Darreichungsform bestehend aus einem Wirkstoff, Erythrit, kristalliner Cellulose und einem Sprengmittel. Weiterhin wird Mannit eingearbeitet und als Sprengmittel quervernetztes Polyvinylpyrrolidon verwendet, sodass eine physikalische Mischung entsteht. Die Tabletten sollen innerhalb von 60 Sekunden in der Mundhöhle zerfallen.

In der Anmeldung WO 2006/029787 wird eine im Mund innerhalb von 60 Sekunden zerfallende Tablette, bestehend aus einem Wirkstoff, einem wasserlöslichen Polyvinylalkohol-Polyethylenglykol-Copolymer, Zucker/Zuckeralkohol (Mannit) und Sprengmittel, beschrieben.

In der WO03041683 werden schnell auflösende Mundtabletten beschrieben enthaltend 30-90% Mannitol, 3-15% eines Sprengmittels ausgewählt aus Croscarmellose, Crospovidon, vorzugsweise weniger als 10 % eines wasserunlöslichen Polymers wie z.B. Ethylcellulose und ein viskositätserhöhendes oder gelbildendes Polymer wie z.B. HPMC, Alginat etc..

Sowohl die US2005244343 also auch die US2005244492 beschreiben schnell auflösende Mundtabletten enthaltend 27-66,25 % eines Zuckeralkohols wie z.B. Mannitol oder Erythriol, 5-10 % eines Sprengmittels ausgewählt aus Stärkederivate, Crospovidon, Croscarmellose, sowie Xanthan als viskositätserhöhendes Polymer. Sie unterscheiden sich von der Anmeldung dadurch, keine wasserunlöslichen filmbildenden Polymere verwendet werden sondern Silikate bzw. Titandioxide.

Aufgabe der vorliegenden Erfindung war es, Kau- und Lutsch-Tabletten zu finden, die gut schmecken, ein angenehmes Mundgefühl hinterlassen und gleichzeitig gut kau- und lutschbar aber doch mechanisch auch sehr stabil sind.

Demgemäß wurde eine pharmazeutische Zubereitung für die Herstellung von Kau- und Lutsch-Tabletten gefunden, enthaltend
A) Agglomerate aus
   a1) 60 - 97 Gew.-% mindestens eines Zuckers oder Zuckeralkohols oder Mischungen davon,
   a2) 1 - 25 Gew.-% einesSprengmittels,
   a3) 1 - 15 Gew.-% von wasserunlöslichen Polymeren,
   a4) 0 - 15 Gew.-% wasserlöslichen Polymeren, und
   a5) 0 - 15 Gew.-% weiterer pharmazeutisch üblichen Hilfsstoffen,
   wobei die Summe der Komponenten a1) bis a5) 100 Gew.-% beträgt, und
B) viskositätserhöhende oder gelbildende Polymere, ausgewählt aus der Gruppe bestehend aus Agar, Alginate, beispielsweise als Natriumalginate, Carrageenan, Guar, Johannisbrotkernmehl, Tara, Aloe, Pectine, Xanthan, Gellan, Dextran, Curdlan, Pullulan, Hydroxypropylmethylcellulose (HPMC), Hydroxypropylcellulose (HPC), Natriumcarboxymethylcellulose, Hydroxyethylcellulose (HEC), Stärke, modifizierte Stärke, Chitosan, Polyacrylsäure- Natrium, Poloxamere und Polyvinylalkohol, gefunden.

Die Zubereitungen können 20 bis 99 , bevorzugt 40 bis 90 Gew.-% an Agglomeraten A) und 0,1 bis 25, bevorzugt 1 bis 15 Gew.-% an Komponenten B) enthalten.

Gewünschtenfalls können auch als Komponenten C) 0 bis 5 Gew.-% Schmiermittel, D) 0,1 bis 50 Gew.-% pharmazeutische Wirkstoffe und als Komponenten E) 0 bis 25 Gew.-% weitere pharmazeutische Hilfsstoffe zugegeben werden.

Dabei beträgt die Summe der Mengen an A),B) und gegebenenfalls C), D) und E) und 100 %.

Der Hilfsstoffanteil A) setzt sich im Einzelnen wie folgt zusammen:
Die pharmazeutischen Zubereitungen enthalten als Komponente a1) 60 bis 97 Gew.-%, bevorzugt 70 bis 95 Gew.-%, besonders bevorzugt 75 bis 93 Gew.-% eines Zuckers, Zuckeralkohols oder Mischungen davon. Als Zucker oder Zuckeralkohole eignen sich Trehalose, Mannit, Erythrit, Isomalt, Maltit, Lactit, Xylit, Sorbit. Die Zucker- oder Zuckeralkoholkomponenten sind bevorzugt feinteilig, mit mittleren Teilchengrößen von 5 bis 100 µm. Gewünschtenfalls können die Teilchengrößen durch Mahlen eingestellt werden. Bevorzugte Teilchengrößen liegen bei 30 bis 50 µm. Es kann sich aber auch empfehlen Teilchengrößen kleiner 30 µm zu verwenden. Ebenso kann es sich empfehlen, Zuckern beziehungsweise Zuckeralkohole einzusetzen, die Mischungen von Fraktionen mit unterschiedlicher Partikelgröße enthalten, bespielsweise Mischungen aus 30 bis 70 gew.-% eienr Korngrößenfraktion mit einer mittleren Teilchengröße von < 30 µm und 30 bis 70 Gew.-% einer Korngrößenfraktion mit einer mittleren Teilchgröße von 30 bis 50 µm. Bevorzugt werden Mannit, Erythrit oder Mischungen davon eingesetzt.

Als Komponente a2) werden Sprengmittel in Mengen von 1 bis 25 Gew.-%, bevorzugt, 2 bis 15 Gew.-%, besonders bevorzugt 3 bis 10 Gew.-%, eingesetzt. Die Sprengmittel sind bevorzugt ausgewählt aus der Gruppe bestehend aus quervernetzten Polyvinylpyrrolidonen, Croscarmellose, Natriumcarboxymethylstärke und L-Hydroxypropylcellulose. Unter Croscarmellose werden erfindungsgemäß die Natrium- und/oder Calciumsalze der quervernetzten Carboxymethycellulose verstanden. Bevorzugte L-Hydroxypropylcellulosen weisen 5 bis 16 % Hydroxypropoxygruppen auf. Besonders bevorzugt sind quervernetzte Polyvinylpyrrolidone. Solche quervernetzten Polyvinylpyrrolidone sind wasserunlöslich, aber nicht filmbildend. Das quervernetzte Polyvinylpyrrolidon kann eine mittlere Teilchengröße von 2 bis 60 µm, bevorzugt kleiner 50 µm, besonders bevorzugt kleiner 30 µm aufweisen.

Als Komponente a3) werden wasserunlösliche Polymere eingesetzt in Mengen von 1 bis 15 Gew.-%, bevorzugt 1 bis 10 Gew.-%, eingesetzt. Bevorzugt sind Polymere, die im pH-Bereich von 1 bis 14 unlöslich sind, also eine bei jedem pH-Wert pHunabhängige Wasserunlöslichkeit aufweisen. Weiterhin eignen sich aber auch Polymere, die bei jedem pH-Wert im pH-Bereich von 6 bis 14 wasserunlöslich sind.

Die Polymere sollen filmbildende Polymere sein. Filmbildend bedeutet in diesem Zusammenhang, dass die Polymere in wässriger Dispersion eine Mindestfilmbildetemperatur von -20 bis +150 °C, bevorzugt 0 bis 100 °C aufweisen.

Geeignete Polymere sind Polyvinylacetat, Ethylcellulose, Methylmethacrylat-Ethylacrylat-Copolymere, Ethylacrylat-Methylmethacrylat-Trimethylammoniumethylmethacrylat-Terpolymere. Butylmethacrylat-Methylmethacrylat-Dimethylaminoethylmethacrylat-Terpolymere Die Acrylat-Methacrylat-Copolymere sind näher beschrieben in der Europäischen Pharmacopoeia als Polyacrylate Dispersion 30%, in der USP als Ammonio Methacrylate Copolymer und in JPE als Aminoalkyl-Methacrylate Copolymer E. Als bevorzugte Komponente c) kommt Polyvinylacetat zum Einsatz. Dieses kann als wässrige Dispersion mit Feststoffgehalten von 10 bis 45 Gew.-% eingesetzt werden. Bevorzugt ist zudem Polyvinylacetat mit einem Molekulargewicht zwischen 100.000 und 1.000.000 Dalton besonders bevorzugt zwischen 200.000 und 800.000 Dalton.

Weiterhin können die Formulierungen als Komponenten a4) wasserlösliche Polymere in Mengen von 0 bis 15 Gew.-% enthalten. Geeignete wasserlösliche Polymere sind beispielsweise Polyvinylpyrrolidone oder Vinylpyrrolidon-Vinylacetat-Copolymere, Polyvinylalkohol-Polyethylenglykol-Pfropfcopolymere, Polyethylenglykole, Ethylenglykol-Propylenglykol-Blockcopolymere.

Gewünschtenfalls können durch Zusatz von pharmazeutisch üblichen Hilfsstoffen (Komponenten a5)) in Mengen von 0 bis 15 Gew.-%, beispielsweise wie Säuerungsmitteln, Puffersubstanzen, Süßstoffen, Aromen, Geschmacksverstärkern und Farbstoffen Geschmack und Aussehen der aus den Formulierungen erhaltenen Tabletten weiter verbessert werden. Folgende Stoffe sind hierbei besonders geeignet: Citronensäure, Weinsäure, Ascorbinsäure, Natriumdihydrogenphosphat, Cyclamat, Saccharin-Na, Aspartam, Menthol, Pfefferminzaroma, Fruchtaromen, Vanillearoma, Glutamat, Riboflavin, Betacarotin, wasserlösliche Farbstoffe, feinteilige Farblacke.
Durch Zusatz von Verdickungsmitteln wie hochmolekularen Polysacchariden kann das Mundgefühl durch Erhöhung der Weichheit und des Volumengefühls zusätzlich verbessert werden.

Weiterhin können als Komponenten a5) auch Tenside zugegeben werden. Als Tenside eignen sich beispielsweise Natriumlaurylsulfat, Dioctylsulfosuccinat, alkoxilierte Sorbitanester wie Polysorbat 80, polyalkoxilierte Derivate von Rizinusöl oder hydriertem Rizinusöl, beispielsweise Cremophor® RH 40, alkoxilierte Fettsäuren, alkoxilierte Hydroxyfettsäuren, alkoxilierte Fettalkohole, Alkalisalze von Fettsäuren und Lecithine.

Weiterhin können zur weiteren Verbesserung des Zerfalls auch feinteilige Pigmente zugegeben werden, weil sie die inneren Grenzflächen erhöhen und somit Wasser schneller in die Tablette eindringen kann. Diese Pigmente wie Eisenoxide, Titandioxid, kolloidale oder gefällte Kieselsäure, Calciumcarbonate, Calciumphosphate müssen natürlich sehr feinteilig sein, ansonsten entsteht wiederum ein körniger Geschmack.

Als Komponenten B) enthalten die Zubereitungen viskositätserhöhende oder gelbildende Polymere oder Mischungen davon. Viskositäterhöhende/ gelbidende Polymere sind gemäß der Erfindung:
Agar, Alginate, beispielsweise als Natriumalginate, Carrageenan, Guar, Johannisbrotkernmehl, Tara, Aloe, Pectine, Xanthan, Gellan, Dextran, Curdlan, Pullulan, Hydroxypropylmethylcellulose (HPMC), Hydroxypropylcellulose (HPC), Natriumcarboxymethylcellulose, Hydroxyethylcellulose (HEC), Stärke, modifizierte Stärke, Chitosan, Polyacrylsäure- Natrium, Poloxamere und Polyvinylalkohol. Bevorzugte Polymere B sind Xanthan, Alginate, HPMC und HPC.

Die erfindungsgemäßen Zubereitungen werden zur Herstellung von Tabletten üblicherweise mit mindestens einem Wirkstoff versetzt.

Als Wirkstoffe können grundsätzlich alle Wirkstoffe eingesetzt werden. Bevorzugt werden die im Folgenden genannten Wirkstoffe, besonders bevorzugt in den genannten Dosierungen, eingesetzt.

Zolmitriptan 2,5 mg, Rizatriptan 5 mg, Diphenhydramin-HCl (geschmacksmaskiert) 20 mg, Brompheniramin 5 mg, Chlorpheniramin 5 mg, Pseudoephedrin (geschmacksmaskiert) 30mg,
Paracetamol (geschmacksmaskiert) 250 mg, Ibuprofen (geschmacksmaskiert) 200 mg, Acetylsalicylsäure 250 mg (geschmacksmaskiert), Hyoscyaminsulfat 0,125 mg, Mirtazapin 15 mg,
Selegelin-HCl 1,25 mg, Ondansetron 4 mg, Olanzapin 5 mg, Clonazepam 1 mg, Cetirizindihydrochlorid 10 mg, Desloratadin 5 mg, Enalaprilmaleat 5 mg, Domperidonmaleat 10 mg, Scopolamin 0,25 mg,
Oxazepam 15 mg, Lorazepam 2,5 mg, Clozapin 25 mg, Dihydroergotaminmesylat 5 mg,
Nicergolin 5 mg, Phloroglucinol 80 mg, Metopimazin 7,5 mg, Triazolam 0,5 mg, Protizolam 0,5 mg,
Tramadol 50 mg, Zolpidemtartrat 5 mg, Cisapride , Risperidon 2 mg, Azithromycin 100 mg (geschmacksmaskiert), Roxithromycin 50 mg (geschmacksmaskiert),
Clarithromycin 125 mg (geschmacksmaskiert), Erythromycinestolat 250 mg (geschmacksmaskiert),
Apomorphin 20 mg, Fentanyl 0,6 mg,
Amphotericin 10 mg, Hydrotalcid 500 mg, Magaldrat 500 mg, Magnesiumsalze 10 - 500 mg, Calciumsalze 10 - 500 mg, Echinacea-Extrakt 80 mg, Aluminiumoxid 200 mg, Magnesiumhydroxid 200 mg, Montelukast 5 mg als Natriumsalz, Dequaliniumchlorid 0,25 mg, Cetylpyridiniumchlorid 1 mg, Buprenorphin 0,4 - 8 mg als HCl, Lorazepam 1 - 5 mg, Dinatriumfluorophosphat 50 - 100 mg, Ambroxol-HCl 20 mg, Benzocain 10 mg, Chlorhexidin-2HCl 5 mg, Flurbiprofen 10 mg,
Es können auch Mischungen von Wirkstoffen eingesetzt werden.

Die genannten Dosierungen stellen die Absolutmengen des jeweiligen Wirkstoffs pro Arzneiform dar. Die Konzentration des Hilfsstoffsanteils und des Wirkstoffanteils in der fertigen Arzneiform richtet sich nach der Größe der Arzneiform. Bei Kau- oder Lutsch-Tabletten sind übliche Tablettengewichte: 100 mg bis 2000 mg.

Die Wirkstoffe können auch mit einem üblichen geschmacksmaskierenden Überzug versehen sein. Geeignete Polymere für solche Coatings sind: Aminoalkyl methacrylate Copolymer E (Eudragit E oder EPO), Polyvinylalkohole in verschiedenen Formulierungen (Opadry AMB, Kollicoat Protect), Kombinationen von wasserunlöslichen Polymeren wie z. B. Polyvinylacetat, Poly(meth)acrylate (Eudragit NE 30 D, NM 30D, RL, RS, RD, Kollicoat EMM 30 D), Ethylcellulose mit wasserlöslichen oder wasserquellbaren nieder- oder hochmolekularen Stoffen (Povidon, Copovidon, HPMC, HPC, Polyethylenglykole, Poloxamere, Polyethylenglykol-Polyvinylalkohol-Pfropfcopolymere, Zucker, Zuckeralkohole, organischen oder anorganische Salze), Kombinationen von wasserlöslichen Filmbildnern (Polyethylenglykol-Polyvinylalkohol-Pfropfcopolymere, HPMC, Polyvinylalkohole) mit Fetten, Wachsen, Fettsäuren und Fettalkoholen.

Die Herstellung der Agglomerate A) kann durch Aufbau-Agglomeration in Mischern, Wirbelschichtgeräten oder Sprühtürmen erfolgen. Dabei werden feste Ausgangsmaterialien und Granulierflüssigkeit zunächst miteinander vermischt und das feuchte Mischgut anschliessend getrocknet. Gemäß der vorliegenden Erfindung wird als Granulierflüssigkeit eine wässrige Dispersion der Komponente a3), des wasserunlöslichen Polymers, eingesetzt.

Gemäß einer Ausführungsform der Erfindung werden ein oder mehrere Wirkstoffe zusammen mit der Zucker oder Zuckeralkohol, Sprengmittel und gewünschtenfalls den Komponenten a4) und a5) in der Wirbelschicht vorgelegt.

Gemäß einer weiteren Ausführungsform werden die Komponenten a1) bis a5) in Abwesenheit eines Wirkstoffs agglomeriert.

Bei der Agglomeration in der Wirbelschicht wird eine wässrige Dispersion des wasserunlöslichen Polymers (Komponente a3)) auf eine wirbelnde Mischung aus Zucker oder Zuckeralkohol, Sprengmittel, gegebenenfalls Wirkstoffen und gegebenenfalls weiteren Komponenten d) und e) gesprüht, wodurch die feinen Teilchen agglomerieren. Die Zulufttemperaturen betragen 30 bis 100°C, die Ablufttemperaturen 20 bis 70°C.

Insbesondere können bei dieser Agglomeration als weitere Komponenten e) die folgenden Hilfsstoffe eingearbeitet werden:
Farbstoffe, Süßstoffe , Aromen, weitere Sprengmittel, Carbonate, Bicarbonate, Säuerungsmittel oder weitere Hilfsstoffe. Die Verwendung von Farbstoffen, wobei anorganische Pigmente, Organische Farblacke oder wasserlösliche Farbstoffe verwendet werden können, führt beispielsweise zu einheitlich durchgefärbten, schnellzerfallenden Tabletten. Geeignete Farbstoffe sind z. B. Riboflavin, Betacarotin, Anthocyane, Carmin, Indigocarmin, Gelborange S, Chinolingelb, Indigotinlack, Brilliant Blau, Sunset Yellow. Diese weiteren Stoffe können entweder in fester Form in die Wirbelbett-Vorlage gegeben werden oder in der Dispersion der komponenten a3) gelöst oder dispergiert werden. Falls die Dispersion unverträglich mit einem solchen Stoff ist, kann dieser in Lösung oder als Suspension auch vor oder nach der Agglomeration mit der Dispersion der Komponenten a3) aufgesprüht werden.

Bei der Herstellung in Sprühtürmen wird vorzugsweise die sogenannte FSD- oder SBD-Technologie (FSD: Fluidized spray drying; SBD: Spray bed drying) eingesetzt. Hierbei wird eine Lösung des Zuckers oder Zuckeralkohols in Wasser zunächst sprühgetrocknet, im unteren Teil des Sprühtrockners oder in einem angeschlossenen Wirbelbett erfolgt die Zugabe von Sprengmittel und die Eindüsung einer wässrigen Dispersion des wasserunlöslichen Polymeren, wodurch die Teilchen agglomerieren. Feine Teilchen können ferner nochmals vor die Sprühdüse der Zucker oder Zuckeralkohollösung geblasen werden und zusätzlich agglomeriert werden. Es ist im Sprühturm, FSD oder SBD auch eine Prozeßführung ausgehend von der kristallinen Form des Zuckers oder Zuckeralkohols möglich. Dabei wird der kristalline Zucker oder Zuckeralkohol am Kopf des Sprühturms oder in den Feingut-Recyclingstrom zugegeben. Durch das Versprühen einer wässrigen Dispersion des wasserunlöslichen Polymeren wird dieser kristalline Feststoff im Turm agglomeriert.

Für den Agglomerationsprozess kann es sich als günstig erweisen, einen mehrstufigen Sprühprozess zu fahren. Zu Anfang wird die Sprührate niedrig gehalten, um ein Überfeuchten der Produktvorlage und damit deren Verkleben zu verhindern. Mit zunehmender Prozessdauer kann die Sprührate erhöht und damit die Agglomerationstendenz erhöht werden. Es ist ebenfalls möglich die Zuluftmenge und/oder -temperatur in entsprechender Weise während des Prozesses anzupassen. Besonders während der Trocknungsphase ist es vorteilhaft, die Zuluftmenge zu reduzieren und damit einem Abrieb der Agglomerate durch eine hohe mechanischen Belastung vorzubeugen.

Als weitere Anpassungsparameter für die Agglomeratgröße kann die Feinheit des Sprühtröpfchens der Bindemittellösung bzw. -dispersion (einstellbar über den Zerstäubungsgasdruck), die Düsengeometrie und Abstand der Düse zum Produktbett angesehen werden. Je feiner und einheitlicher gesprüht wird, desto feiner und einheitlicher resultieren die Agglomerate. Je weiter die Düse vom Produktbett entfernt ist, desto schlechter ist das Agglomerationsverhalten.

Weiterhin kann die Agglomeration auch in einem Mischer durch eine kontinuierlich geführte Mischaggregation erfolgen. Eine solche kontinuierlich geführte Form der Mischaggregation ist die sogenannte "Schugi-Granulation". Dabei werden in einem kontinuierlich arbeitenden vertikal angeordneten Hochgeschwindigkeitsmischer feste Ausgangsmaterialien und die das wasserunlösliche Polymer enthaltende Granulierflüssigkeit intensiv miteinander vermischt (siehe auch M. Bohnet, "Mechanische Verfahrenstechnik", Wiley VCH Verlag, Weinheim 2004, S. 198 ff.)

Gemäß einer besonderen Ausführungsform wird das Sprengmittel in der wässrigen Dispersion des wasserunlöslichen Polymers suspendiert.

Die so erhaltenen Agglomerate weisen eine mittlere Teilchengröße von 100 - 600 µm, bevorzugt 120 - 500 µm und besonders bevorzugt von 140 - 400 µm auf.
Das wasserunlösliche, filmbildende Polymer dient dabei als Agglomerationsmittel, um die feinen Zucker oder Zuckeralkoholkristalle, die Wirkstoffpartikel und das Sprengmittel sowie gebenenfalls weitere Hilfsstoffe zu Teilchen zu agglomerieren.

Gemäß einer weiteren Ausführungsform der Erfindung kann auch zunächst der Hilfsstoffanteil A) agglomeriert werden und anschliessend in einem weiteren Granulationsschritt eine Agglomeration von A) mit einem oder mehreren Wirkstoffen erfolgen.

Gemäß einer Variante dieser Ausführungsform werden der agglomerierte Hilfsstoffanteil und Wirkstoff in der Wirbelschicht vorgelegt und mittels einer Bindemittellösung granuliert. Die Bindemittellösung enthält als Bindemittel dabei ein wasserlösliches Polymer ausgewählt aus der Gruppe der Komponenten d). Es handelt sich bevorzugt um eine wässrige Lösung. Die Bindemittelkonzentration kann 5 bis 40 Gew.-% betragen. Bevorzugte Bindemittel sind wasserlösliche Polyvinylpyrrolidone mit K-Werten nach Fikentscher von 10 bis 100, insbesonder K30, Vinylpyrrolidon-Vinylacetat-Copolymere aus 30 bis 70 Gew.-% N-Vinypyrrolidon, bevorzugt 40 bis 60 Gew.-%, und 30 bis 70 Gew.-%, bevorzugt 40 bis 60 Gew.-% Vinylacetat, weiterhin Polyvinylalkohole sowie Pfropfcopolymere aus Polyethylenglykol und Polyvinylalkohol.

Gemäß einer zweiten Variante wird der agglomerierte Hilfsstoffanteil in der Wirbelschicht vorgelegt und der Wirkstoffanteil in die oben beschriebene Bindemittellösung eingearbeitet.

Gemäß einer weiteren Variante, bei der das wasserlösliche Bindemittel (Komponente a4)) obligatorisch in dem voragglomerierten Hilfsstoffanteil bereits enthalten ist, vorzugsweise in Mengen von 0,5 bis 10 Gew.-%, wird als Granulierflüssigkeit Wasser ohne polymeres Bindemittel eingesetzt. Andere Hilfsstoffe aus der Gruppe der Komponenten a5) können dabei dem für die Granulierung verwendeten Wasser zugegeben werden. Weiterhin kann es sich empfehlen einen der genannten Zucker oder Zuckeralkohole dem Wasser zuzugeben.

Für alle diese Varianten der Ausführungsform, bei der zunächst der Hilfsstoffanteil agglomeriert wird, werden ansonsten die gleichen oben beschriebenen Vorrichtungen und Prozessparameter angewandt wie bei der gleichzeitigen Agglomeration von Hilfsstoffanteil und Wirkstoffanteil.

Die erfindungsgemäßen Formulierungen können vorteilhaft für die Herstellung von Kau- und Lutsch-Tabletten verwendet werden.

Für die Herstellung von Tabletten können die üblichen Verfahren verwendet werden, wobei die Direkttablettierung und die Walzenkompaktierung besondere Vorteile bieten. Aufgrund der besonderen Eigenschaften der erfindungsgemäßen Formulierungen werden in der Regel nur Wirkstoff, erfindungsgemäße Formulierung und ein Schmiermittel benötigt. Die Tablettenrezeptur ist somit sehr einfach, sehr reproduzierbar und das Verfahren leicht zu validieren.

Ferner besitzen die erfindungsgemäßen Zubereitungen außergewöhnlich gute Fließfähigkeiten und Verpressbarkeiten, die zu mechanisch sehr stabilen Tabletten führen. Die Bruchfestigkeit der mit Hilfe der erfindungsgemäßen pharmazeutischen Formulierungen erzeugten Tabletten beträgt > 50N. Häufig liegen die Bruchfestigkeiten oberhalb von 80 N, selbst unter Verwendung schwer pressbarer Wirkstoffe. Die Friabilitäten betragen < 0,2 %. Beschädigungen beim üblichen Tablettenhandling treten somit nicht auf.

Die erfindungsgemäßen Formulierungen sind damit bei Lagerung sehr stabil und behalten ihr ansprechendes Aussehen.

### Beispiele

Der durch Wirbelschichtagglomeration hergestellte schnellzerfallende Hilfsstoff wurde mit Wirkstoffen und vikositätserhöhendenden/ gelbildenden Polymeren sowie 2,0 Gew.% Schmiermittel (Mg- Stearat) in einem Turbulamischer für 10min gemischt. Anschließend wurden diese Mischungen auf einer vollinstrumentierten Exzenterpresse (Korsch XP1) tablettiert (30Hub/min).

**Tabelle 1: Rezepturzusammensetzung A bis D in Gew.-%**

| | A | B | C | D |
|---|---|---|---|---|
| Mannitol (d0,5:36µm] | 85,7 | 81,2 | 76,2 | 77,2 |
| Kollidon CL-SF | 4,8 | 4,4 | 4,2 | 4,4 |
| Kollicoat SR30D (Feststoff) | 4,8 | 4,4 | 4,2 | 4,4 |
| Alginat | 2,5 | - | 5,0 | - |
| Xanthural 75 | - | 5,0 | - | 10,0 |
| Coffein (feines Pulver) | - | - | 8,4 | - |
| Flurbiprofen | | 3,0 | | 2,0 |
| Dequaliniumchlorid | 0,2 | | | |
| Magnesiumstearat | 2,0 | 2,0 | 2,0 | 2,0 |

Zur Tablettierung der Mischungen A-D wurde ein 10mm Stempel (biplan, facettiert) verwendet. Die Tabletten wurden auf ein Gewicht von 300mg und eine Bruchfestigkeit von 40-50N gepresst.

Die Tabletten der Mischung D wurden mit einer Presskraft von 19kN auf ein Gewicht von 1000mg und einem Durchmesser von 16mm gepresst.

Die Tabletten wurden hinsichtlich Bruchfestigkeit (Tablettentester HT-TMB-CI-12 F, Fa. Kraemer), Zerfallszeit in Phosphatpuffer pH 7,2 (Zerfallstester ZT 74, Erweka) und Freisetzungsgeschwindigkeit in Magensaft (Freisetzungsgerät, Erweka) untersucht.

**Tabelle 2: Tabletteneigenschaften Formulierung A-D**

| | Tablettierdaten | Tablettenparameter | |
|---|---|---|---|
| | Presskraft [kN] | Bruchfestigkeit [N] | Zerfallszeit [min:s] |
| A | 4,3 | 38 | 10:17 |
| B | 5,8 | 50 | 42:06 |
| C | 5,6 | 52 | 19:14 |
| D | 18,9 | 132 | 100:01 |

## Patentansprüche

1. Kau- und Lutschtabletten, enthaltend
A) einen agglomerierten Hitfsstoffanteil aus
a1) 60 - 97 Gew.-% Zucker oder Zuckeralkohole,
a2) 1 - 25 Gew.-% eines Sprengmittels ausgewählt aus der Gruppe beste hend aus Crospovidon, Croscarmellose, Natriumcarboxymethylstärke und L-Hydroxypropylcellulose,
a3) 1 -15 Gew.-% wasseruntösilche, filmbildende Polymere
a4) 0 -15 Gew.-% wasserlösliche Polymere, und
a5) 0 -15 Gew.-% weitere pharmazeutisch übliche Hilfsstoffe, wobei die Summe der Komponenten a1) bis a5) 100 Gew.-% beträgt,
B) mindestens ein viskositätserhöhendes oder gelbildendes Polymer ausgewählt aus der Gruppe bestehend aus Agar, Alginate, beispielsweise als Natriumalginate, Carrageenan, Guar, Johannisbrotkernmehl, Tara, Aloe, Pectine, Xanthan, Gellan, Dextran, Curdlan, Pullulan, Hydroxypropylmethylcellulose (HPMC). Hydroxypropylcellulose (HPC), Natriumcarboxymethylcellulose. Hydroxyethylcellulose (HEC), Stärke, modifizierte Stärke, Chitosan, Polyacrylsäure- Natrium, Poloxamere und Polyvinylalkohol.

2. Kau- und Lutschtabletten nach Anspruch 1 enthaltend eine Komponente B) in Mengen von 0,1 bis 25 Gew.-%.

3. Kau- und Lutschtabletten nach einem der Ansprüche 1 bis 2, enthaltend
A) einen agglomerierten Hilfsstoffanteil aus
a1) 60 - 97 Gew.-% Zucker oder Zuckeralkohole,
a2) 1 - 25 Gew.% eines Sprengmittels ausgewählt aus der Gruppe beste hend aus Crospovidon, Croscarmellose, Natriumcarboxymethylstärke und L-Hydroxypropylcellulose,
a3) 1 -15 Gew.% wasserunlösliche, filmbildende Polymere
a4) 0 - 15 Gew.% wasserlösliche Polymere, und
a5) 0 - 15 Gew.-% weitere pharmazeutisch übliche Hilfsstoffe, wobei die Summe der Komponenten a1) bis a5) 100 Gew.% beträgt,
B) mindestens ein viskositätserhöhendes oder gelbildendes Polymer ausgewählt aus der Gruppe bestehend aus Agar, Alginate, beispielsweise als Natriumalginate, Carrageenan, Guar, Johannisbrotkernmehl, Tara, Aloe, Pectine, Xanthan, Gellan, Dextran, Curdlan, Pullulan, Hydroxypropylmethylcellulose (HPMC), Hydroxypropylcellulose (HPC), Natriumcarboxymethylcellulose, Hydroxyethylcellulose (HEC), Stärke, modifizierte Stärke. Chitosan. Polyacrylsäure- Natrium, Poloxamere und Polyvinylalkohol,
C) 0 bis 10 Gew.-%, bezogen auf die Gesamtmenge aller Komponenten eines Schmiermittels, und
D) mindestens einen pharmazeutischen Wirkstoff.

4. Kau- und Lutschtabletten nach einem der Ansprüche 1 bis 3, enthaltend zusätzlich zu den Komponente A) bis D) weitere pharmazeutische Hilfsstoffe E).

5. Kau- und Lutschtabletten nach einem der Ansprüche 1 bis 4, enthaltend als Komponente E ein Sprengmittel.

6. Kau- und Lutschtabletten nach einem der Ansprüche 1 bis 5, enthaltend Schmiermittel D in Mengen von 0,2 bis 5%.

7. Kau- und Lutschtabletten nach einem der Ansprüche 1 bis 6 enthaltend als Schmiermittel D Magnesiumstearat oder Stearinsäure.

8. Kau- und Lutschtabletten nach einem der Ansprüche 1 bis 7, enthaltend als Zuckeralkohol Mannit oder. Erythrit oder Mischungen davon.

9. Kau- und Lutschtabletten nach einem der Ansprüche 1 bis 8, enthaltend ein Croscarmellose als Natrium- oder Calcium-Salz.

10. Kau- und Lutschtabletten nach einem der Ansprüche 1 bis 8 enthaltend eine L-Hydroxypropylcellulose mit 5 bis 16 % Hydroxypropxygruppen.

11. Kau- und Lutschtabletten nach einem der Ansprüche 1 bis 8, enthaltend als Sprengmittel Crospovidon

12. Kau- und Lutschtabletten nach einem der Ansprüche 1 bis 11 , enthaltend als wasserunlösliches filmbildendes Polymer Polyvinylacetat.

13. Kau- und Lutschtabletten nach einem der Ansprüche 1 bis 12 wobei das wasserunlösliche filmbildende Polymer Polyvinylacetat in Form einer wässrigen Dispersion eingesetzt wird.

14. Kau- und Lutschtabletten nach einem der Ansprüche 1 bis 13, wobei als wasserlösliches Polymer Polyvinylpyrrolidon verwendet wird.

15. Kau- und Lutschtabletten nach einem der Ansprüche 1 bis 14, wobei als weitere pharmazeutisch übliche Stoffe Säuerungsmittel, Süßstoffe, Aromen, Geschmacksverstärker, Farbstoffe, Verdickungsmittel, Tenside und feinteilige Pigmente verwendet werden.

16. Kau- und Lutschtabletten nach einem der Ansprüche 1 bis 15, enthaltend Agglomerate A)
aus
a1) 70 - 95 Gew.-% Zucker oder Zuckeralkoholen
a2) 2-15 Gew.-% eines Sprengmittels,
a3) 1 -10 Gew.% wasserunlösliche, filmbildende Polymere
a4) 0 - 2 Gew.-% wasserlösliches Polyvinylpyrrolidon, und
a5) 0 - 15 Gew.-% weiteren pharmazeutisch übliche Hilfsstoffe.

17. Kau- und Lutschtabletten nach einem der Ansprüche 1 bis 16, enthaltend Agglomerate A) aus
a1) 75 - 95 Gew.-% Mannit oder Erythrit oder einer Mischung davon,
a2) 3 - 10 Gew.% eines Sprengmittels ,
a3) 1-10 Gew.-% Polyvinylacetat,
a4) 0 - 2 Gew.-% wasserlösliches Polyvinylpyrrolidon, und
a5)0 - 15 Gew.-% weiteren pharmazeutisch übliche Hilfsstoffe.

18. Kau- und Lutschtabletten nach einem der Ansprüche 1 bis 17, enthaltend als Komponente
B) Alginate oder Xanthan oder Hydroxypropylmethylcellulose.

## Claims

1. A chewable or suckable tablet comprising
A) an agglomerated excipient content composed of
a1) 60-97% by weight of sugar or sugar alcohols,
a2) 1-25% by weight of a disintegrant, selected from the group consisting of crospovidone, croscarmellose, sodium carboxymethylstarch and L-hydroxypropylcellulose,
a3) 1-15% by weight of water-insoluble, film-forming polymers
a4) 0-15% by weight of water-soluble polymers and
a5) 0-15% by weight of further pharmaceutically customary excipients the total of the components a1) to a5) being 100% by weight,
B) at least one viscosity-increasing or gel-forming polymer selected from the group consisting of agar, alginates, for example as sodium alginates, carrageenan, guar, locust bean gum, tara, aloe, pectins, xanthan, gellan, dextran, curdlan, pullulan, hydroxypropylmethylcellulose (HPMC), hydroxypropylcellulose (HPC), sodium carboxymethylcellulose, hydroxyethylcellulose (HEC), starch, modified starch, chitosan, polyacrylic acid sodium, poloxamers and polyvinyl alcohol.

2. The chewable or suckable tablet according to claim 1 comprising a component B) in amounts of from 0.1 to 25% by weight.

3. The chewable or suckable tablet according to either of claims 1 and 2, comprising
A) an agglomerated excipient content composed of
a1) 60-97% by weight of sugar or sugar alcohols,
a2) 1-25% by weight of a disintegrant, selected from the group consisting of crospovidone, croscarmellose, sodium carboxymethylstarch and L-hydroxypropylcellulose,
a3) 1-15% by weight of water-insoluble, film-forming polymers
a4) 0-15% by weight of water-soluble polymers and
a5) 0-15% by weight of further pharmaceutically customary excipients, the total of the components a1) to a5) being 100% by weight,
B) at least one viscosity-increasing or gel-forming polymer selected from the group consisting of agar, alginates, for example as sodium alginates, carrageenan, guar, locust bean gum, tara, aloe, pectins, xanthan, gellan, dextran, curdlan, pullulan, hydroxypropylmethylcellulose (HPMC), hydroxypropylcellulose (HPC), sodium carboxymethylcellulose, hydroxyethylcellulose (HEC), starch, modified starch, chitosan, polyacrylic acid sodium, poloxamers and polyvinyl alcohol,
C) from 0 to 10% by weight, based on the total amount of all components, of a lubricant, and
D) at least one active pharmaceutical ingredient.

4. The chewable or suckable tablet according to any of claims 1 to 3, comprising, in addition to components A) to D), further pharmaceutical excipients E).

5. The chewable or suckable tablet according to any of claims 1 to 4, comprising a disintegrant as component E.

6. The chewable or suckable tablet according to any of claims 1 to 5, comprising lubricant D in amounts of from 0.2 to 5%.

7. The chewable or suckable tablet according to any of claims 1 to 6, comprising magnesium stearate or stearic acid as lubricant D.

8. The chewable or suckable tablet according to any of claims 1 to 7, comprising mannitol or erythritol or mixtures thereof as sugar alcohol.

9. The chewable or suckable tablet according to any of claims 1 to 8, comprising a croscarmellose as sodium or calcium salt.

10. The chewable or suckable tablet according to any of claims 1 to 8, comprising an L-hydroxypropylcellulose having 5 to 16% hydroxypropoxy groups.

11. The chewable or suckable tablet according to any of claims 1 to 8, comprising crospovidone as disintegrant.

12. The chewable or suckable tablet according to any of claims 1 to 11, comprising polyvinyl acetate as water-insoluble film-forming polymer.

13. The chewable or suckable tablet according to any of claims 1 to 12, where the water-insoluble film-forming polymer polyvinyl acetate is employed in the form of an aqueous dispersion.

14. The chewable or suckable tablet according to any of claims 1 to 13, where polyvinylpyrrolidone is used as water-soluble polymer.

15. The chewable or suckable tablet according to any of claims 1 to 14, where acidifiers, sweeteners, flavors, flavor enhancers, colorants, thickeners, surfactants and finely divided pigments are used as further pharmaceutically customary substances.

16. The chewable or suckable tablet according to any of claims 1 to 15, comprising agglomerates A) composed of
a1) 70-95% by weight of sugar or sugar alcohols
a2) 2-15% by weight of a disintegrant,
a3) 1-10% by weight of water-insoluble, film-forming polymers
a4) 0-2% by weight of water-soluble polyvinylpyrrolidone, and
a5) 0-15% by weight of further pharmaceutically customary excipients.

17. The chewable or suckable tablet according to any of claims 1 to 16, comprising agglomerates A) composed of
a1) 75-95% by weight of mannitol or erythritol or a mixture thereof,
a2) 3-10% by weight of a disintegrant,
a3) 1-10% by weight of polyvinyl acetate,
a4) 0-2% by weight of water-soluble polyvinylpyrrolidone, and
a5) 0-15% by weight of further pharmaceutically customary excipients.

18. The chewable or suckable tablet according to any of claims 1 to 17, comprising alginates or xanthan or hydroxypropylmethylcellulose as component B).

## Revendications

1. Pastilles à croquer et à sucer, contenant
A) une proportion d'adjuvant agglomérée constituée par
a1) 60-97% en poids de sucre ou d'alcools de sucre,
a2) 1-25% en poids d'un agent de désintégration choisi dans le groupe constitué par la crospovidone, la croscarmellose, le carboxyméthylamidon de sodium et la L-hydroxypropylcellulose,
a3) 1-15% en poids de polymères filmogènes insolubles dans l'eau
a4) 0-15% en poids de polymères solubles dans l'eau, et
a5) 0-15% en poids d'autres adjuvants pharmaceutiquement usuels, la somme des composants a1) à a5) valant 100% en poids,
B) au moins un polymère augmentant la viscosité ou gélifiant, choisi dans le groupe constitué par l'agar, les alginates, par exemple sous forme d'alginates de sodium, le carraghénane, la gomme guar, la farine de noyaux de caroube, la gomme tara, l'aloe, la pectine, la gomme xanthane, la gomme gellane, le dextrane, le curdlan, le pullulane, l'hydroxypropylméthylcellulose (HPMC), l'hydroxypropylcellulose (HPC), la carboxyméthylcellulose sodique, l'hydroxyéthylcellulose (HEC), l'amidon, l'amidon modifié, le chitosane, le poly(acide acrylique) sodique, les poloxamères et le poly(alcool vinylique).

2. Pastilles à croquer et à sucer selon la revendication 1, contenant un composant B) en des quantités de 0,1 à 25% en poids.

3. Pastilles à croquer et à sucer selon l'une quelconque des revendications 1 à 2, contenant
A) une proportion d'adjuvant agglomérée constituée par
a1) 60-97% en poids de sucre ou d'alcools de sucre,
a2) 1-25% en poids d'un agent de désintégration choisi dans le groupe constitué par la crospovidone, la croscarmellose, le carboxyméthylamidon de sodium et la L-hydroxypropylcellulose,
a3) 1-15% en poids de polymères filmogènes insolubles dans l'eau
a4) 0-15% en poids de polymères solubles dans l'eau, et
a5) 0-15% en poids d'autres adjuvants pharmaceutiquement usuels,
la somme des composants a1) à a5) valant 100% en poids,
B) au moins un polymère augmentant la viscosité ou gélifiant, choisi dans le groupe constitué par l'agar, les alginates, par exemple sous forme d'alginates de sodium, le carraghénane, la gomme guar, la farine de noyaux de caroube, la gomme tara, l'aloe, la pectine, le xanthane, la gomme gellane, le dextrane, le curdlan, le pullulane, l'hydroxypropylméthylcellulose (HPMC), l'hydroxypropylcellulose (HPC), la carboxyméthylcellulose sodique, l'hydroxyéthylcellulose (HEC), l'amidon, l'amidon modifié, le chitosane, le poly(acide acrylique) sodique, les poloxamères et le poly(alcool vinylique),
C) 0 à 10% en poids, par rapport à la quantité totale de tous les composants, d'un lubrifiant, et
D) au moins une substance active pharmaceutique.

4. Pastilles à croquer et à sucer selon l'une quelconque des revendications 1 à 3, contenant en plus des composants A) à D) d'autres adjuvants pharmaceutiques E).

5. Pastilles à croquer et à sucer selon l'une quelconque des revendications 1 à 4, contenant, comme composant E, un agent de désintégration.

6. Pastilles à croquer et à sucer selon l'une quelconque des revendications 1 à 5, contenant le lubrifiant D en des quantités de 0,2 à 5%.

7. Pastilles à croquer et à sucer selon l'une quelconque des revendications 1 à 6, contenant, comme lubrifiant D, du stéarate de magnésium ou de l'acide stéarique.

8. Pastilles à croquer et à sucer selon l'une quelconque des revendications 1 à 7, contenant, comme alcool de sucre, du mannitol ou de l'érythritol.

9. Pastilles à croquer et à sucer selon l'une quelconque des revendications 1 à 8, contenant une croscarmellose sous forme de sel de sodium ou de calcium.

10. Pastilles à croquer et à sucer selon l'une quelconque des revendications 1 à 8 contenant une L-hydroxypropylcellulose présentant 5 à 16% de groupes hydroxypropxy.

11. Pastilles à croquer et à sucer selon l'une quelconque des revendications 1 à 8, contenant, comme agent de désintégration, de la crospovidone.

12. Pastilles à croquer et à sucer selon l'une quelconque des revendications 1 à 11 , contenant, comme polymère filmogène insoluble dans l'eau, du poly(acétate de vinyle).

13. Pastilles à croquer et à sucer selon l'une quelconque des revendications 1 à 12, le polymère filmogène insoluble dans l'eau, le poly(acétate de vinyle), étant utilisé sous forme d'une dispersion aqueuse.

14. Pastilles à croquer et à sucer selon l'une quelconque des revendications 1 à 13, de la polyvinylpyrrolidone étant utilisée comme polymère soluble dans l'eau.

15. Pastilles à croquer et à sucer selon l'une quelconque des revendications 1 à 14, des agents acidifiants, des édulcorants, des arômes, des exhausteurs de goût, des colorants, des épaississants, des agents tensioactifs et des pigments finement divisés étant utilisés comme autres substances pharmaceutiquement usuelles.

16. Pastilles à croquer et à sucer selon l'une quelconque des revendications 1 à 15, contenant des agglomérats A) constitués par
a1) 70-95% en poids de sucre ou d'alcools de sucre,
a2) 2-15% en poids d'un agent de désintégration,
a3) 1-10% en poids de polymères filmogènes insolubles dans l'eau
a4) 0-2% en poids de polyvinylpyrrolidone soluble dans l'eau, et
a5) 0-15% en poids d'autres adjuvants pharmaceutiquement usuels.

17. Pastilles à croquer et à sucer selon l'une quelconque des revendications 1 à 16, contenant des agglomérats A) constitués par
a1) 75-95% en poids de mannitol ou d'érythritol ou d'un mélange de ceux-ci,
a2) 3-10% en poids d'un agent de désintégration,
a3) 1-10% en poids de poly(acétate de vinyle),
a4) 0-2% en poids de polyvinylpyrrolidone soluble dans l'eau, et
a5) 0-15% en poids d'autres adjuvants pharmaceutiquement usuels.

18. Pastilles à croquer et à sucer selon l'une quelconque des revendications 1 à 17, contenant, comme composant
B) des alginates ou de la gomme xanthane ou de l'hydroxypropylméthylcellulose.
